(19) Europäisches Patentamt
European Patent Office
Office européen des brevets

(11) **EP 4 489 652 B1**

(12) **EUROPEAN PATENT SPECIFICATION**

(45) Date of publication and mention
of the grant of the patent:
**28.01.2026  Bulletin 2026/05**

(21) Application number: **23710435.1**

(22) Date of filing: **20.02.2023**

(51) International Patent Classification (IPC):
*A61B 5/287* (2021.01)      *A61B 5/339* (2021.01)
*A61B 5/367* (2021.01)      *A61B 5/00* (2006.01)
*A61B 18/00* (2006.01)

(52) Cooperative Patent Classification (CPC):
**A61B 5/725; A61B 5/287; A61B 5/339;**
**A61B 5/367; A61B 5/6858; A61B 5/7435;**
**A61B 18/1492;** A61B 2018/00357;
A61B 2018/00577; A61B 2018/00839;
A61B 2090/065

(86) International application number:
**PCT/IB2023/051521**

(87) International publication number:
**WO 2023/170494 (14.09.2023 Gazette 2023/37)**

(54) **SMART CARDIAC ELECTROPHYSIOLOGICAL (EP) MAP**

INTELLIGENTE ELEKTROPHYSIOLOGISCHE (EP) HERZKARTE

CARTE ÉLECTROPHYSIOLOGIQUE (EP) CARDIAQUE INTELLIGENTE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **07.03.2022   US 202263317117 P**
          **09.02.2023   US 202318107751**

(43) Date of publication of application:
**15.01.2025   Bulletin 2025/03**

(73) Proprietor: **Biosense Webster (Israel) Ltd.**
**2066717 Yokneam (IL)**

(72) Inventors:
• **WEISS, Shaked**
**2066717 Yokneam (IL)**

• **YARNITSKY, Jonathan**
**2066717 Yokneam (IL)**
• **COHEN, Assaf**
**2066717 Yokneam (IL)**
• **NOVOGRODSKY, Ben Ami**
**2066717 Yokneam (IL)**
• **SEGEV, Meytal**
**2066717 Yokneam (IL)**

(74) Representative: **Carpmaels & Ransford LLP**
**One Southampton Row**
**London WC1B 5HA (GB)**

(56) References cited:
**US-A1- 2021 386 355**

**Description**

**CROSS-REFERENCE TO RELATED APPLICATION**

This application claims the benefit of U.S. Provisional Patent Application 63/317,117, filed March 7, 2022.

**FIELD OF THE DISCLOSURE**

The present disclosure relates generally to cardiac electrophysiological (EP) mapping, and particularly to automation of generation of data points for cardiac EP maps.

**BACKGROUND OF THE DISCLOSURE**

Automatic processing of catheter-acquired EP signals was previously described in the patent literature. For example, U.S. Patent Application Publication 2009/0099468 describes a method, an apparatus, and a computer program product for automated processing of intracardiac electrophysiological data. The method comprises the steps of: recording electrogram data and corresponding spatial location data of an electrode recording the electrogram data, the recorded electrogram data comprising a plurality of beats; defining at least one reference channel containing a reference beat for determining temporal locations and against which beats of the recorded electrogram data are compared; examining the recorded electrogram data and defining a temporal location for each beat of the recorded electrogram data; creating an index of the temporal locations and other information of the beats within the recorded electrogram data; analyzing in real-time at least one electrophysiological feature of the recorded electrogram data suggestive of a physiological condition; and providing an updated index wherein the other information comprises results of the analysis.

As another example, U.S. Patent 10,376,221 describes automatic creation of multiple electroanatomic maps. Cardiac electrograms are recorded in a plurality of channels. Beats are classified automatically into respective classifications according to a resemblance of the morphologic characteristics of the beats to members of a set of templates. Respective electroanatomic maps of the heart are generated from the classified beats.

In US2021386355A1, there is described a system and method for detecting a mapping annotation for an electrophysiological (EP) mapping system. The system includes a processor comprising a machine learning algorithm configured to receive a first heartbeat at an identified cardiac spatial location including a first set of attributes information corresponding to the first heartbeat; receive a second heartbeat at the identified cardiac spatial location including a second set of attributes information corresponding to the second heartbeat; compare the first set of attributes information with the second set of attributes information; and determine which of the first heartbeat and the second heartbeat has optimal characteristics based on the compared attribute information.

The present disclosure will be more fully understood from the following detailed description of the examples thereof, taken together with the drawings in which:

**BRIEF DESCRIPTION OF THE DRAWINGS**

Fig. 1 is a schematic, pictorial illustration of a system for electrophysiological (EP) mapping and ablation, in accordance with an example of the present disclosure;

Fig. 2 is a flow chart that schematically illustrates a method for considering a candidate data point based on automatic quality scoring of the data point, in accordance with an example of the present disclosure;

Fig. 3 is a schematic illustration of a graphical user interface (GUI) of the electrophysiological (EP) mapping system of Fig. 1, the GUI including a scale for setting a quality scoring threshold, in accordance with an example of the present disclosure; and

Fig. 4 is a schematic illustration of an informative layer of an EP map, the layer spatially indicating EP map quality according to quality score of the automatically accepted data points by the process of Fig. 2, in accordance with an example of the present disclosure.

**DETAILED DESCRIPTION OF EXAMPLES**

OVERVIEW

Probe-based (e.g., catheter-based) cardiac diagnostic and therapeutic systems may measure multiple intra-cardiac electrophysiological (EP) signals, such as electrograms (EGM), during an invasive procedure. Such systems acquire multiple intra-cardiac signals using one or more electrodes (e.g., multiple electrodes) that are fitted at the distal end of the probe. For example, to reduce the time required to EP map a heart chamber, e.g., to acquire EGM signals of the chamber, a catheter carrying a large number of electrodes (e.g., 256), such as a basket catheter, may be used. In many cases, the analysis of such a large number EP signals acquired in parallel is done, for example, to generate data points used to generate an EP map in continuous mode (automatically), in order to enable processing such a vast amount of EP information.

The generation of the data points typically involves applying rejection criteria (e.g., the use of "filtering" to avoid using "erroneous" data points, such as those that are irrelevant or too noisy) at any given time. The physician performing the procedure therefore needs to set "adequate" filtering criteria and to monitor the filtration process in order to ensure that its settings produce a sufficient quality (e.g., relevant, stable) of data points (e.g., for an EP map).

Thus, acquired data points are typically subjected to filtration (e.g., rejection criteria) to remove data points that may be incorrect (e.g., unstable) or irrelevant (e.g., acquired from the blood pool instead of the chamber surface). When acquired data points are subjected to multiple filters each having individual thresholds, a failure of a point to pass any one filter causes the point to be rejected, even if it might be acceptable if not high-quality, all things considered. For large total numbers of data points acquired during an EP mapping session, which, using modern multi-electrode mapping catheters, number in the tens of thousands, it is difficult for a physician to tell whether the filter setting levels are reasonable, e.g., whether too many or too few data points are being rejected. Furthermore, in conventional maps, once a data point is acquired, it becomes "permanent," with no ability to replace the data point with a higher quality point, thus preventing the map from reaching the highest potential quality achievable within the data capable of being acquired.

Examples of the present disclosure that are described herein provide algorithms and visualizations for a high level (e.g., based mainly on clinical input) and automated selection of data points to, for example, generate and update an EP map in real time. In some examples, each newly received data point is scored by a processor with a disclosed quality score. The disclosed algorithm (called SMARTMAP) prioritizes the scores of high-quality data points over lower-quality data points when constructing (e.g., visualizing) an EP map, by swapping data-point selections according to their quality score. To this end the processor is configured to replace a previously-selected data point within a given unit volume defined below with a newly-acquired data point in the given unit volume, in response to finding that the quality score of the newly-acquired data point is larger than the quality score of the previously-selected data point.

The aforementioned quality score of a data point, also called herein a "smart index," is an output of a weight function which considers several data point parameters obtained during a preprocessing stage (e.g., annotations) of an intra-cardiac waveform. The weighted parameters include, for example, cycle length, signal amplitude, stability of local activation time (LAT), electrode stability and level of contact touch with tissue during acquisition, among others, as described below. In some examples, the processor rates each new data point with a normalized quality score ranging between 0 and 1.

In some examples, the process of data point selection and EP map updating according to newly selected data points is done in real time while continuous acquisition is in progress. The disclosed SmartMap technique reduces physician work load (i.e., user workflow) by removing data point filtering steps, which results in simpler and faster data point acquisition and quick EP map settings. User involvement and decision making are therefore limited to high-level settings, such as arrhythmia type and quality-score threshold, all performed via a high-level GUI.

One example of high-level visualization provided comprises a layer of the EP map, sometimes referred to as a "heat map", that spatially indicates EP map quality according to the quality score (i.e., "smart index") of the graded data points. This layer graphically indicates regions of a cardiac chamber to guide the physician for mapping catheter insertion. Where the quantity of data points is too low (e.g., spatially sparse), the physician can collect additional data points so as to improve the EP map quality in such regions. Another high-level visualization is an adjustable scale for the aforementioned user-selected threshold value of the quality score, so the user can fine-tune the EP map quality by changing a quality scoring threshold below which candidate data points are not considered.

SYSTEM DESCRIPTION

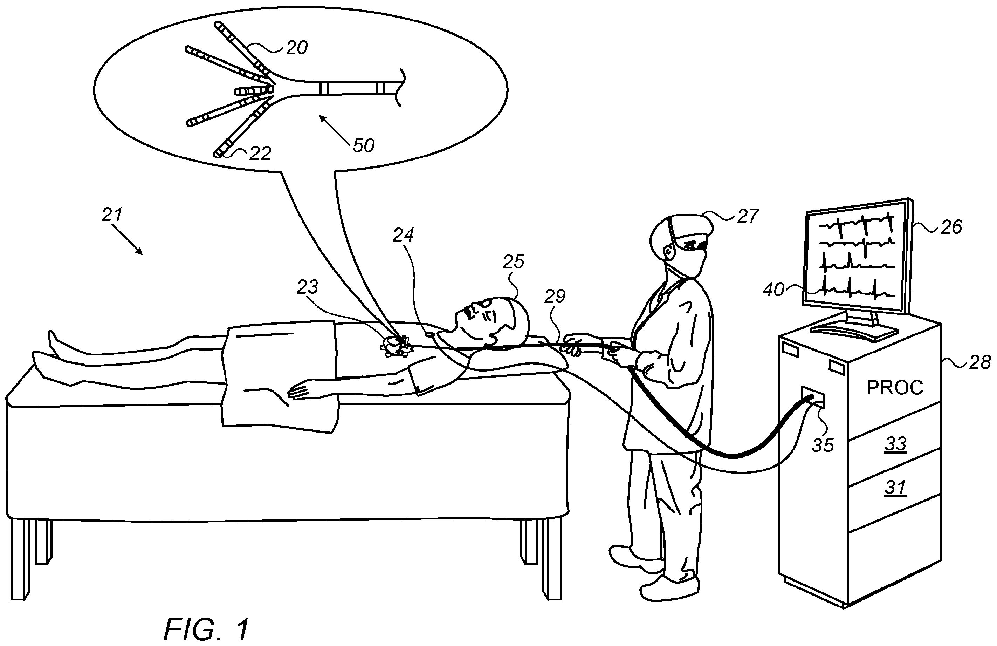

Fig. 1 is a schematic, pictorial illustration of a system 21 for electrophysiological (EP) mapping and ablation, in accordance with an example of the present disclosure. Fig. 1 depicts a physician 27 using an EP mapping catheter 29 to perform an EP mapping of a heart 23 of a patient 25. Catheter 29 comprises, at its distal end, a multi-channel electrode array 50 comprising one or more arms 20, each of which is coupled to mapping-electrodes 22. During the mapping procedure, electrodes 22 acquire and/or inject signals from and/or to the tissue of heart 23. In particular, electrodes 22 acquire intracardiac EP signals, such as unipolar and/or bipolar electrograms (e.g., electrograms 40 shown on a display 26 of system 21).

Catheter 29 may be further used to perform an ablation, such as radiofrequency (RF) or irreversible electroporation (IRE).

The respective locations of mapping-electrodes 22 inside heart 23 (i.e., where the electrograms are measured) are also tracked, such that a processor 28 may link each acquired electrogram with the location at which the signal was acquired to, for example, generate a data point for an EP map. System 21 externally senses electrical position signals using a plurality of external electrodes 24 coupled to the body of patient 25. For example, three external electrodes 24 may be coupled to the patient's chest, and another three external electrodes may be coupled to the patient's back. For ease of illustration, only one external electrode is shown in Fig. 1.

An example of a system capable of using the sensed electrical position signals to track the locations of mapping-electrodes 22 inside heart 23 of the patient is the CARTO®3 system (produced by Biosense Webster, Irvine, California). The CARTO®3 system uses a position tracking method named Advanced Current Location (ACL), which is described in detail in U.S. Patent 8,456,182.

A data acquisition module 33 receives the multiple electrogram conveyed to an electrical interface 35 over a wire link that runs in catheter 29. Processor 28 of system 21 receives these cardiac signals via an electrical interface 35, and, after processing these into data points, uses the disclosed algorithm to sort the data points based on, for example, different predefined arrhythmia types, e.g., atrial fibrillations, tachycardias, flutter, and others. In this way physician 27 doesn't need to operate a menu of data filters in order to determine the suitability of the acquired data points for use in an EP map. It is noted that the disclosed algorithms herein merely represent some exemplary algorithms among many that could be used within the scope of the appended claims.

Processor 28 stores the acquired data points in a memory 31 for further analysis, such as for constructing an EP map. An example of an EP map that processor 38 may produce from automatically sorted data points is a local activation time (LAT) map. A method for generating an LAT map is described in U.S. Patent 9,050,011, which is assigned to the assignee of this application.

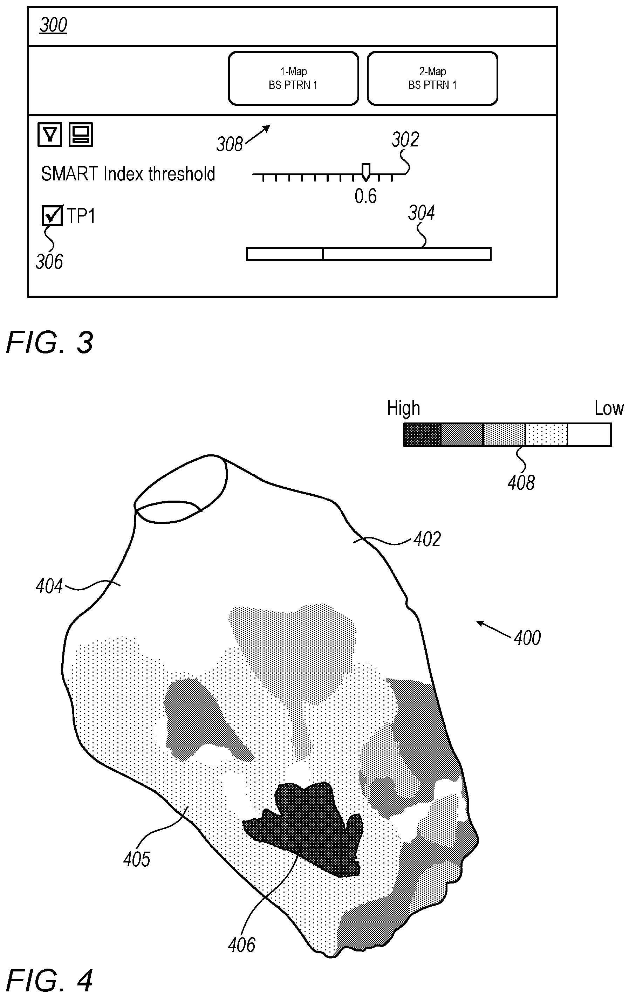

Processor 28 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 28 runs a dedicated algorithm that enables processor 28 to perform the steps described in Fig. 3.

The example illustration shown in Fig. 1 is chosen purely for the sake of conceptual clarity. Other types of multi-electrode sensing geometries, such as of the Lasso® catheter (produced by Biosense Webster) or a basket catheter, may also be employed. Additionally, contact sensors may be fitted at the distal end of electro-anatomical catheter 29 to transmit data indicative of the physical quality of electrode contact with tissue.

## METHOD FOR AUTOMATIC QUALITY SCORING OF DATA POINTS

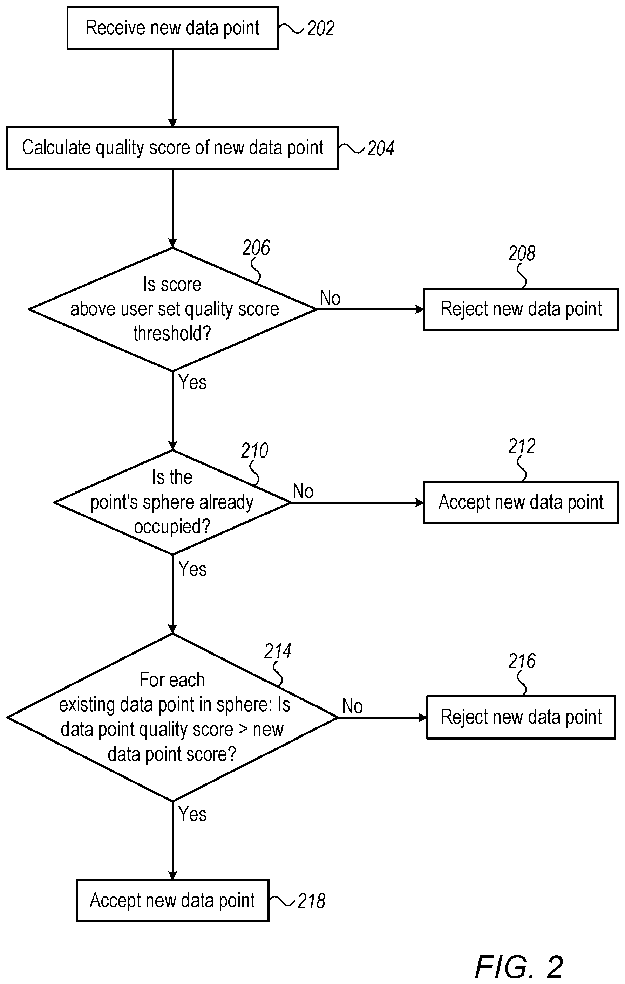

Fig. 2 is a flow chart that schematically illustrates an example method for considering (i.e., rejecting or accepting) a candidate data point based on automatic quality scoring of the data point, in accordance with an example of the present disclosure. The algorithm, according to the presented example, carries out a process that begins with processor 28 receiving (e.g., uploading) a new data point, at a new data point receiving step 202. Received data points are aggregated by the processor (e.g., stored in memory 31) as part of a point cloud that may be used in generating an EP map or for other uses.

At quality score calculation step 204, processor 28 calculates the quality score of the data point, according to Eq. 1 below.

Then, processor 28 compares the calculated quality score to a user-set quality score threshold, at a comparison step 206. If the calculated quality score is lower than the threshold, processor 28 rejects the new data point, at data point rejection step 208.

If the calculated quality score is higher than the threshold, processor 28 checks if a unit volume of predefined size that encompass the newly received data point (e.g., a sphere of predefined radius such as 1 mm), is occupied by one or more existing data points, at data points existence checking step 210.

If the answer is no, meaning that the newly received data point is the only data point in the predefined unit volume, processor 28 accepts the data point, at data point accepting step 212.

If the answer is yes, processor 28 checks if the quality score of the newly received data point is higher than those of the existing data points in the unit volume, at a quality score value comparison step 314.

If the answer is no, processor 28 rejects the new data point, at data point rejection step 316. If the answer is yes, processor 28 accepts the data point, at data point accepting step 312. The accepted data point (from either step 212 or step 218) can be used by the processor to update an existing EP map..

## QUALITY SCORE OF DATA POINT IN SMARTMAP

As noted above, the smart index is an outcome of a weight function which weighs several parameters of a data point, as listed, for example, in Table I below. Each candidate data point is rated with a normalized score (also called smart index) ranging between 0 and 1.

An exemplary equation of data point smart index is:

$$\text{Eq. 1} \quad \frac{\sum_{n=1}^{N} parameter's\ weight_n * parameter's\ score_n}{\sum_{n=1}^{N} parameter's\ weight_n},$$

where N is the total number of parameters (see Table I of parameters below), with each parameter given a weight, e.g., a score from 1 to 5. Again, the parameter's normalized score is a number ranging between 0 and 1.

Table I

| Parameter Name | Parameter Weight | Parameter Score |
|---|---|---|
| Pattern matching | 5 | (-1) to 1<br>According to the PM correlation score |
| CL | 2 | $\|Current\ CL - median\ CL\| = \Delta$<br>$\sigma^2 = CL\ variance$<br>$score = \begin{cases} 1 - \dfrac{\Delta}{\sigma^2}, & current\ CL\ is\ in\ range \\ 0, & current\ CL\ is\ out\ range \end{cases}$ |
| LAT stability | 3 | $\|Current\ LAT - Prev.\ beat\ LAT\| = \Delta$<br>$score = \begin{cases} 1 - \dfrac{\Delta}{12}, & \Delta \leq 12 \\ 0, & \Delta > 12 \end{cases}$ |
| Complex data point (e.g., LAM, CFAE) | 5 | 1/0 |
| TPI | 4 | 1 (touch)/0 (no touch or unknown) |
| Position stability | 3 | $\|Current\ position - Prev.\ beat\ position\| \equiv \Delta$<br>$score = \begin{cases} 1 - \dfrac{\Delta}{10}, & \Delta \leq 10 \\ 0, & \Delta > 10 \end{cases}$ |
| Respiration | 5 | 1/0 (in/out respiration threshold) |
| Location (CPM) | 2 | 0.2 |
| SNR | 2 | $score = \begin{cases} \dfrac{SNR}{10}, & SNR \leq 10 \\ 1, & SNR > 10 \end{cases}$ |
| $\left\|\dfrac{dV}{dt}\right\|$ | 2 | $score = \begin{cases} \left\|\dfrac{dV}{dt}\right\|/5, & \left\|\dfrac{dV}{dt}\right\| \leq 5 \\ 1, & \left\|\dfrac{dV}{dt}\right\| > 5 \end{cases}$ |

In Table I, complex fractionated atrial electrograms (CFAE) and late activation mapping (LAM indicate complex EP signal behavior, such as those from a scarred tissue region. Because this deserves focus from the clinician, this distinction may therefore receive a high weight, at least for some arrhythmias.

In Table I, pattern matching is a test of data point consistency over cardiac cycles (a value of correlation between cardia cycles).

CL stands for cardia cycle length.

TPI stands for touch pressure index (or contact pressure index), which estimates contact force of a catheter electrode with a tissue wall during acquisition of the data point.

Position stability is a measure of electrode position consistency during acquisition over two or more cardiac cycles.

Respiratory weights adversely impact respiration on acquisition, such as introducing noise to signals.

Location CPM is a measure of the aforementioned ACL position tracking technique on electrode location consistency during acquisition.

SNR stands for the signal to noise ratio, which, if low, indicates a less robust acquisition. $\left|\dfrac{dV}{dt}\right|$ reflects for the sharpness of the signal deflections.

Table I is brought by way of example only. Other parameter lists may be different.

GRAPHICAL USER INTERFACE TOOLS FOR SMARTMAP

Fig. 3 is a schematic illustration of a graphical user interface (GUI) 300 of electrophysiological (EP) mapping system 21 of Fig. 1, GUI 300 including a scale 302 for setting a quality scoring threshold, in accordance with an example of the present disclosure.

GUI 300 further includes a status bar 304 on the level of correlation results of pattern matching. A low value may mean sub-optimal acquisition conditions, which may indicate to a physician about a need to improve acquisition conditions in order to produce more meaningful EP data.

A TPI check box 306 allows a user to include a TPI weight, a time-consuming step in the process of accepting data points, e.g., for updating an EP map.

Finally, region 308 presents active EP maps, through which the user can toggle in order to, for example, adjust thresholds according to map type or arrhythmia type in question.

Fig. 3 is an example brought solely for the sake of describing an example. An actual GUI is typically far more elaborate, including, for example, numerous icons and graphics that are omitted herein for simplicity of presentation.

Fig. 4 is a schematic illustration of an informative layer 400 of an EP map, layer 400 spatially indicating quality of the EP map according to quality score of the automatically accepted data points by the process of Fig. 2, in accordance with an example of the present disclosure. The shown map depicts anatomy 402 of a left ventricle being EP mapped with a catheter.

A scale 408 indicates to a user a quality score of data points at different regions of mapped anatomy 402. As seen, some regions (404) have not yet been mapped. Some regions (e.g., highlighted regions 405) are mapped at various medium quality score levels of data points. Finally, some regions, such as region 406, are mapped with high quality score data points. Using layer 400, which is a type of heat map of quality score of accepted and used data points, which a physician performing the EP mapping can use to direct the catheter to regions where EP map quality can be improved.

It will be appreciated that the examples described above are cited by way of example, and that the present disclosure is not limited to what has been particularly shown and described hereinabove.

**Claims**

1. A system (21) for generating an electrophysiological (EP) map, the system comprising:

   a display (26); and
   a processor (28), configured to:

      receive multiple EP data points comprising respective locations and EP values, generated from signals acquired by one or more electrodes (22) of a catheter (29) that are in contact with tissue of a cardiac chamber;
      score the received data points with respective quality scores;
      for a given unit volume of the EP map, select, from among the data points whose locations fall in the unit volume, a data point with a highest quality score, for use in generating the EP map; and
      visualize the EP map to a user, on the display;
      and **characterised in that** the processor is configured to calculate the quality score according to a user-selected type of arrhythmia.

2. The system according to claim 1, wherein the quality score comprises a weighted scoring of at least two of (i) signal pattern matching, (ii) annotated cycle length, (iii) LAT stability, (iv) complex/simple data point flagging, (v) electrode contact pressure index, (vi) electrode position stability (vii) respiratory flax, (vii) signal SNR, and (viii) sharpness of signal deflection.

3. The system according to claim 1, wherein the processor is configured to replace a previously-selected data point of the given unit volume with a newly-acquired data point in the given unit volume, in response to finding that the quality score of the newly-acquired data point is larger than the quality score of the previously-selected data point.

4. The system according to claim 1, wherein the processor is further configured to generate and present to the user on the display a layer (400) of the EP map that spatially indicates a quality of the EP map according to a quality score of the selected data points.

5. The system according to claim 1, wherein the processor is further configured to display a graphical user interface (GUI) (300) that enables the user to set a threshold of the quality score, and to reject any data point whose quality score is below the threshold.

6. A computer-implemented method for generating an electrophysiological (EP) map, the method comprising:

receiving multiple EP data points comprising respective locations and EP values, generated from signals acquired by one or more electrodes (22) of a catheter (29) that are in contact with tissue of a cardiac chamber;
scoring the received data points with respective quality scores;
for a given unit volume of the EP map, selecting, from among the data points whose locations fall in the unit volume, a data point with a highest quality score, for use in generating the EP map; and
visualizing the EP map to a user, on a display;
and **characterised in that** the quality score is calculated according to a user-selected type of arrhythmia.

7. The method according to claim 6, wherein the quality score comprises a weighted scoring of at least two of (i) signal pattern matching, (ii) annotated cycle length, (iii) LAT stability, (iv) complex/simple data point flagging, (v) electrode contact pressure index, (vi) electrode position stability (vii) respiratory flax, (vii) signal SNR, and (viii) sharpness of signal deflection.

8. The method according to claim 6, and comprising replacing a previously-selected data point of the given unit volume with a newly-acquired data point in the given unit volume, in response to finding that the quality score of the newly-acquired data point is larger than the quality score of the previously-selected data point.

9. The method according to claim 6, and comprising generating and presenting to the user on the display a layer (400) of the EP map that spatially indicates a quality of the EP map according to a quality score of the selected data points.

10. The method according to claim 6, and comprising displaying a graphical user interface (GUI) (300) that enables the user to set a threshold of the quality score, and to reject any data point whose quality score is below the threshold.

**Patentansprüche**

1. System (21) zum Erzeugen einer elektrophysiologischen (EP) Karte, das System umfassend:

eine Anzeige (26); und
einen Prozessor (28), der konfiguriert ist zum:

Empfangen von mehreren EP-Datenpunkten, umfassend jeweilige Positionen und EP-Werte, die aus Signalen erzeugt werden, die durch eine oder mehrere Elektroden (22) eines Katheters (29) erfasst werden, die mit Gewebe einer Herzkammer in Kontakt stehen;
Bewerten der empfangenen Datenpunkte mit jeweiligen Qualitätsbewertungen;
für ein gegebenes Einheitsvolumen der EP-Karte, Auswählen, aus den Datenpunkten, deren Positionen in das Einheitsvolumen fallen, eines Datenpunkts mit einer höchsten Qualitätsbewertung zur Verwendung beim Erzeugen der EP-Karte; und
Visualisieren der EP-Karte einem Benutzer auf der Anzeige;
und **dadurch gekennzeichnet, dass** der Prozessor konfiguriert ist, um die Qualitätsbewertung gemäß einer benutzergewählten Art von Arrhythmie zu berechnen.

2. System nach Anspruch 1, wobei der Qualitätswert eine gewichtete Bewertung von mindestens zwei von (i) Signalmusterabgleich, (ii) annotierter Zykluslänge, (iii) LAT-Stabilität, (iv) Kennzeichnung komplexer/einfacher Datenpunkte, (v) Elektrodenkontaktdruckindex, (vi) Elektrodenpositionsstabilität, (vii) Atemflachs, (vii) Signal-SNR und (viii) Schärfe einer Signalauslenkung umfasst.

3. System nach Anspruch 1, wobei der Prozessor konfiguriert ist, um einen zuvor ausgewählten Datenpunkt des gegebenen Einheitsvolumens durch einen neu erfassten Datenpunkt in dem gegebenen Einheitsvolumen zu ersetzen, als Reaktion darauf, dass festgestellt wird, dass die Qualitätsbewertung des neu erfassten Datenpunkts größer ist als die Qualitätsbewertung des zuvor ausgewählten Datenpunkts.

4. System nach Anspruch 1, wobei der Prozessor ferner konfiguriert ist, um eine Schicht (400) der EP-Karte, die eine Qualität der EP-Karte gemäß einer Qualitätsbewertung der ausgewählten Datenpunkte räumlich angibt, zu erzeugen und dem Benutzer auf der Anzeige darzustellen.

5. System nach Anspruch 1, wobei der Prozessor ferner konfiguriert ist, um eine grafische Benutzeroberfläche (GUI) (300) anzuzeigen, die es dem Benutzer ermöglicht, eine Schwelle der Qualitätsbewertung festzulegen und jeden Datenpunkt abzulehnen, dessen Qualitätsbewertung unter der Schwelle liegt.

6. Computer-implementiertes Verfahren zum Erzeugen einer elektrophysiologischen (EP) Karte, das Verfahren umfassend:

Empfangen von mehreren EP-Datenpunkten, umfassend jeweilige Positionen und EP-Werte, die aus Signalen erzeugt werden, die durch eine oder mehrere Elektroden (22) eines Katheters (29) erfasst werden, die mit Gewebe einer Herzkammer in Kontakt stehen;
Bewerten der empfangenen Datenpunkte mit jeweiligen Qualitätsbewertungen;
für ein gegebenes Einheitsvolumen der EP-Karte, Auswählen, aus den Datenpunkten, deren Positionen in das Einheitsvolumen fallen, eines Datenpunkts mit einer höchsten Qualitätsbewertung zur Verwendung beim Erzeugen der EP-Karte; und
Visualisieren der EP-Karte einem Benutzer auf einer Anzeige;
und **dadurch gekennzeichnet, dass** die Qualitätsbewertung gemäß einer benutzergewählten Art von Arrhythmie berechnet wird.

7. Verfahren nach Anspruch 6, wobei der Qualitätswert eine gewichtete Bewertung von mindestens zwei von (i) Signalmusterabgleich, (ii) annotierter Zykluslänge, (iii) LAT-Stabilität, (iv) Kennzeichnung komplexer/einfacher Datenpunkte, (v) Elektrodenkontaktdruckindex, (vi) Elektrodenpositionsstabilität, (vii) Atemflachs, (vii) Signal-SNR und (viii) Schärfe einer Signalauslenkung umfasst.

8. Verfahren nach Anspruch 6, und umfassend das Ersetzen eines zuvor ausgewählten Datenpunkts des gegebenen Einheitsvolumens durch einen neu erfassten Datenpunkt in dem gegebenen Einheitsvolumen als Reaktion darauf, dass festgestellt wird, dass die Qualitätsbewertung des neu erfassten Datenpunkts größer ist als die Qualitätsbewertung des zuvor ausgewählten Datenpunkts.

9. Verfahren nach Anspruch 6, und umfassend das Erzeugen und Darstellen einer Schicht (400) der EP-Karte, die eine Qualität der EP-Karte gemäß einer Qualitätsbewertung der ausgewählten Datenpunkte räumlich angibt.

10. Verfahren nach Anspruch 6, und umfassend das Anzeigen einer grafischen Benutzeroberfläche (GUI) (300), die es dem Benutzer ermöglicht, eine Schwelle der Qualitätsbewertung festzulegen und jeden Datenpunkt abzulehnen, dessen Qualitätsbewertung unter der Schwelle liegt.

**Revendications**

1. Système (21) de génération d'une carte électrophysiologique (EP), le système comprenant :

un dispositif d'affichage (26) ; et
un processeur (28), configuré pour :

recevoir de multiples points de données EP comprenant des emplacements et des valeurs EP respectifs, générés à partir de signaux acquis par une ou plusieurs électrodes (22) d'un cathéter (29) qui sont en contact avec le tissu d'une chambre cardiaque ;

attribuer aux points de données reçus des scores de qualité respectifs ;

pour un volume unitaire donné de la carte EP, sélectionner, parmi les points de données dont les emplacements se situent dans le volume unitaire, un point de données ayant un score de qualité élevé, pour une utilisation dans la génération de carte EP ; et

visualiser la carte EP pour un utilisateur, sur le dispositif d'affichage ;

et **caractérisé en ce que** le processeur est configuré pour calculer le score de qualité en fonction d'un type d'arythmie sélectionné par l'utilisateur.

2. Système selon la revendication 1, dans lequel le score de qualité comprend une notation pondérée d'au moins deux parmi (i) une correspondance de motif de signal, (ii) une longueur de cycle annotée, (iii) une stabilité de LAT, (iv) un signalement de point de données complexe/simple, (v) un indice de pression de contact d'électrode, (vi) une stabilité de position d'électrode, (vii) un lin respiratoire, (vii) un RSB de signal, et (viii) une netteté de déflexion du signal.

3. Système selon la revendication 1, dans lequel le processeur est configuré pour remplacer un point de données précédemment sélectionné du volume unitaire donné par un point de données nouvellement acquis dans le volume unitaire donné, en réponse à la constatation que le score de qualité du point de données nouvellement acquis est supérieur au score de qualité du point de données précédemment sélectionné.

4. Système selon la revendication 1, dans lequel le processeur est en outre configuré pour générer et présenter à l'utilisateur sur le dispositif d'affichage une couche (400) de la carte EP qui indique spatialement une qualité de la carte EP en fonction d'un score de qualité des points de données sélectionnés.

5. Système selon la revendication 1, dans lequel le processeur est en outre configuré pour afficher une interface utilisateur graphique (GUI) (300) qui permet à l'utilisateur de régler un seuil du score de qualité et de rejeter tout point de données dont le score de qualité est inférieur au seuil.

6. Procédé mis en œuvre par ordinateur pour générer une carte électrophysiologique (EP), le procédé comprenant :

la réception de multiples points de données EP comprenant des emplacements et des valeurs EP respectifs, générés à partir de signaux acquis par une ou plusieurs électrodes (22) d'un cathéter (29) qui sont en contact avec le tissu d'une chambre cardiaque ;

l'attribution de scores de qualité respectifs aux points de données reçus ;

pour un volume unitaire donné de la carte EP, la sélection, parmi les points de données dont les emplacements se situent dans le volume unitaire, d'un point de données ayant un score de qualité le plus élevé, pour une utilisation dans la génération de la carte EP ; et

la visualisation de la carte EP pour un utilisateur, sur un dispositif d'affichage ;

et **caractérisé en ce que** le score de qualité est calculé en fonction d'un type d'arythmie sélectionné par l'utilisateur.

7. Procédé selon la revendication 6, dans lequel le score de qualité comprend une notation pondérée d'au moins deux parmi (i) une correspondance de motif de signal, (ii) une longueur de cycle annotée, (iii) une stabilité de LAT, (iv) un signalement de point de données complexe/simple, (v) un indice de pression de contact d'électrode, (vi) une stabilité de position d'électrode, (vii) un lin respiratoire, (vii) un RSB de signal, et (viii) une netteté de déflexion du signal.

8. Procédé selon la revendication 6, et comprenant le remplacement d'un point de données précédemment sélectionné du volume unitaire donné par un point de données nouvellement acquis dans le volume unitaire donné, en réponse à la constatation que le score de qualité du point de données nouvellement acquis est supérieur au score de qualité du point de données précédemment sélectionné.

9. Procédé selon la revendication 6, et comprenant la génération et la présentation à l'utilisateur sur le dispositif d'affichage d'une couche (400) de la carte EP qui indique spatialement une qualité de la carte EP en fonction d'un score de qualité des points de données sélectionnés.

10. Procédé selon la revendication 6, et comprenant l'affichage d'une interface utilisateur graphique (GUI) (300) qui permet à l'utilisateur de régler un seuil du score de qualité et de rejeter tout point de données dont le score de qualité est inférieur au seuil.

FIG. 1

Receive new data point 〜*202*

↓

Calculate quality score of new data point 〜*204*

↓

*206*

Is score above user set quality score threshold? — No → Reject new data point *208*

↓ Yes

*210*

Is the point's sphere already occupied? — No → Accept new data point *212*

↓ Yes

*214*

For each existing data point in sphere: Is data point quality score > new data point score? — No → Reject new data point *216*

↓ Yes

Accept new data point 〜*218*

*FIG. 2*

FIG. 3

FIG. 4

**EP 4 489 652 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63317117
- US 20090099468
- US 10376221 B

- US 2021386355 A1
- US 8456182 B
- US 9050011 B